(19) Europäisches Patentamt
European Patent Office
Office européen des brevets

(11) Veröffentlichungsnummer: **0 115 274**
**B1**

(12) # EUROPÄISCHE PATENTSCHRIFT

(45) Veröffentlichungstag der Patentschrift: **05.11.86**

(21) Anmeldenummer: **84100129.0**

(22) Anmeldetag: **09.01.84**

(51) Int. Cl.[4]: **C 07 D 303/04**, C 11 B 9/00, A 23 L 1/226, C 07 C 35/52, C 07 C 13/48, C 07 C 13/465

(54) **Epoxide von bicyclischen Verbindungen, Verfahren zu deren Herstellung und Riech- und/oder Geschmackstoffkompositionen mit einem Gehalt an solchen Verbindungen.**

(30) Priorität: **13.01.83 CH 158/83**

(43) Veröffentlichungstag der Anmeldung: **08.08.84 Patentblatt 84/32**

(45) Bekanntmachung des Hinweises auf die Patenterteilung: **05.11.86 Patentblatt 86/45**

(84) Benannte Vertragsstaaten: **CH DE FR GB LI NL**

(56) Entgegenhaltungen:
**DE-A-2 064 921**
**FR-A-2 189 353**

**TETRAHEDRON LETTERS, Nr. 49, 1976, Seiten 4521-4524, Oxford, GB. J.E.L. McDONALD et al.: "The synthesis of 2,2,8-trimethyltricyclo(6,2,2,0,1,6)dodec-5ene; a rearrangement product of thujopsene"**

(73) Patentinhaber: **L. GIVAUDAN & CIE Société Anonyme, CH-1214 Vernier-Genève (CH)**

(72) Erfinder: **Helmlinger, Daniel, Dr., Tichelrütistrasse 18, CH-8044 Gockhausen (CH)**
Erfinder: **Pesaro, Mario, Dr., Wiesliacher 55, CH-8053 Zürich (CH)**

(74) Vertreter: **Urech, Peter, Dr., Grenzacherstrasse 124 Postfach 3255, CH-4002 Basel (CH)**

EP 0 115 274 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Riech- und/oder Geschmackstoffe. Es handelt sich dabei um bicyclische Epoxide der allgemeinen Formel

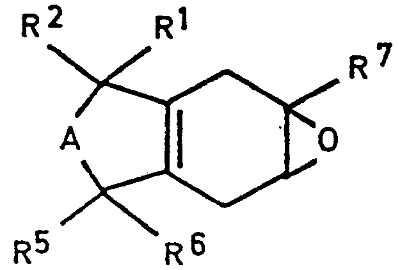

I

worin A für 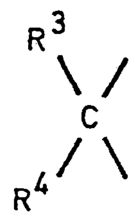 oder 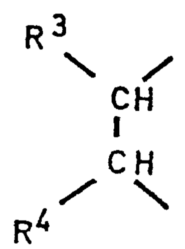 steht und die

steht und die Symbole $R^1$, $R^2$, $R^5$ und $R^6$ H, $CH_3$, $C_2H_5$ oder $-CH(CH_3)_2$ darstellen, wobei aber H, $C_2H_5$ oder $-CH(CH_3)_2$ jeweils nur einmal vorhanden ist, und die Symbole $R^3$, $R^4$ und $R^7$ H oder $CH_3$ darstellen.

Die neuen Verbindungen umfassen also sowohl Epoxide mit dem Octahydronaphthalin (Iα)- als auch solche mit dem Tetrahydroindan (Iβ)-Gerüst

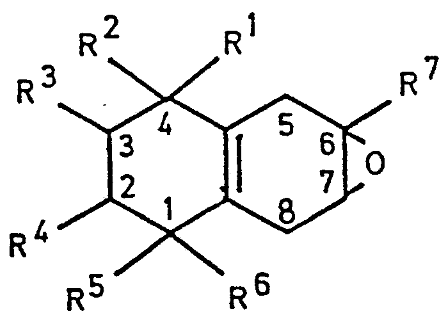

Iα

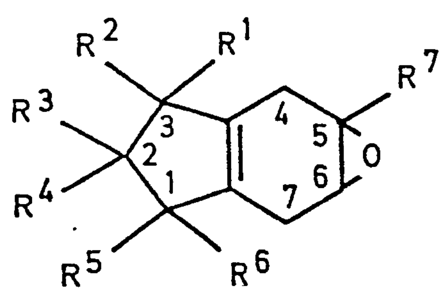

I β

Die Erfindung betrifft ferner ein Verfahren zur Herstellung der Verbindungen der Formel I.

Dieses Verfahren ist dadurch gekennzeichnet, dass man ein bicyclisches 1,4-Cyclohexadien der Formel

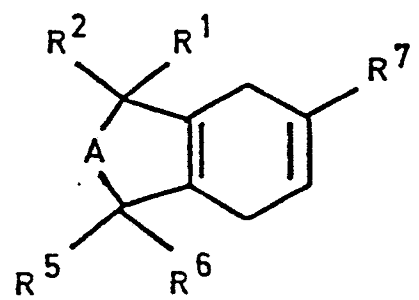

II

worin A und $R^1$ bis $R^7$ obige Bedeutung besitzen, epoxidiert.

Von den an sich bekannten Epoxidierungsmethoden für C=C-Doppelbindungen sind insbesondere folgende besonders gut geeignet:

1) Herstellung von I aus II über die α-Halogen-hydrine

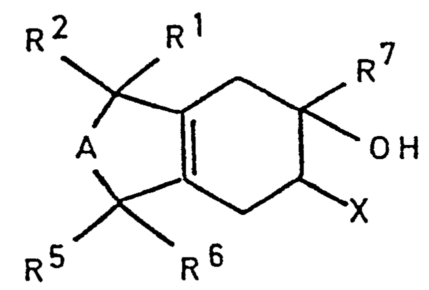

III

worin A und die Reste $R^1$ bis $R^7$ obige Bedeutung haben und X Chlor, Brom oder Jod bedeutet,

z.B. durch Addition einer unterhalogenigen Säure HOX an die drei- bzw. zweifach substituierte Doppelbindung von II (vgl. Houben-Weyl, "Methoden der Organischen Chemie", Band VI, 1a, Teil 1 (1979) Seite 564), gefolgt durch Dehydrohalogenierung der α-Halogen-hydrine III durch Basenbehandlung (vgl. z.B. "Organic Functional Group Preparations", S.R. Sandler and W. Karo, Acad. Press 1968, S. 109 sowie "Organic Reactions in Steroid Chemistry" Vol. II, Ed. J. Fried and J.A. Edwards, Van Nostrand Reinhold Co., 1972, S. 15).

2) Behandlung der 1,4-Cyclohexadiene II mit einer organischen Persäure, wie z.B. Peressig- oder Perbenzoesäure in einem inerten Lösungsmittel, wie z.B. Dichlormethan (vgl. "Oxidation in Organic Chemistry" Ed. W.S. Trahanovsky Part C, Acad. Press 1978, S. 225).

3) Behandlung der 1,4-Cyclohexadiene II mit einem Alkylhydroperoxid, wie z.B. tert. Butyl- oder Aethylbenzolhydroperoxid, in Gegenwart eines Metallkatalysators, wie z.B. einer Molybdän- oder Vanadium-Verbindung. Die Reaktion wird zweckmässigerweise in einem inerten Lösungsmittel, wie z.B. Benzol oder 1,2-Dichloräthan, bei Temperaturen zwischen 80° und 120°C durchgeführt (vgl. "Metal catalyzed Oxidations of Organic Compounds", R.A. Sheldon and J.K. Kochi, Acad. Press 1981, S. 275).

Bei der Epoxidierung der 1,4-Cyclohexadiene II können ausser den Epoxiden I auch die damit isomeren Epoxide

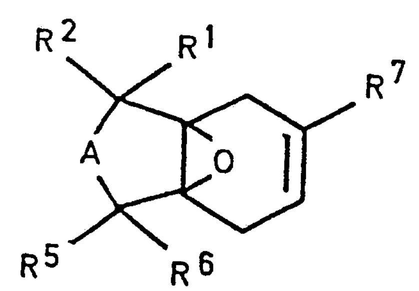

IV

worin A und die Reste $R^1$ bis $R^7$ obige Bedeutung besitzen,

entstehen. Diese Epoxide IV sind die Reaktionsprodukte der durch die Substituenten $R^1$ bis $R^6$ sterisch gehinderten, tetrasubstituierten Doppelbindung mit dem Oxidationsreagens. Das Verhältnis der Epoxide I/IV

hängt von der Struktur des bicyclischen 1,4-Cyclohexadiens II und von der angewandten Epoxidierungsmethode ab. Die Epoxidierung mit Alkylhydroperoxid/Metallkatalysator (Methode 3) ist sehr regioselektiv und liefert überwiegend bis ausschliesslich die Epoxide I; somit ist diese Methode die bevorzugte Epoxidierungsmethode.

Nach dem erfindungsgemässen Verfahren können die Verbindungen I in Abhängigkeit von der Art der Substituenten $R^1$ bis $R^7$ als Diastereomeren- bzw. Struktur-Isomerengemische anfallen. Die Trennung dieser Gemische in die einzelnen Komponenten kann z.B. mittels Gaschromatographie oder Säulenchromatographie durchgeführt werden. Aus wirtschaftlichen Gründen werden aber bevorzugterweise die Gemische verwendet.

Die Herstellung der 1,4-Cyclohexadienderivate II erfolgt durch Reduktion der entsprechenden bekannten Benzolderivate

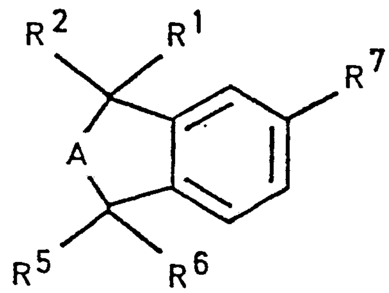

V

worin A und die Reste $R^1$ bis $R^7$ obige Bedeutung besitzen,

nach an sich bekannter Art, z.B. mittels Alkali- bzw. Erdalkalimetallen in flüssigem Ammoniak/Alkohol nach Birch oder mittels Alkali- bzw. Erdalkalomatallen im System Alkylamin/Alkohol nach Benkeser, oder elektrochemisch an Metallkathoden. (Vgl. Houben-Weyl, "Methoden der Organischen Chemie", Band V, 1b (1972) S. 613).

Die Reduktion nach Birch wird bei tiefer Temperatur (z.B. -78° bis -33° C) in flüssigem Ammoniak, das Zusätze eines Alkohols, wie z.B. Aethanol enthält, durchgeführt. Die Reduktion nach Benkeser wird z.B. in Methyl-, Aethylamin, Aethylendiamin, etc. in Gegenwart eines Alkohols wie Aethanol, Isopropanol, Isoamylalkohol usw. durchgeführt.

Als Metalle eignen sich besonders Natrium und Lithium. Ein Zusatz von Diäthyläther oder Tetrahydrofuran begünstigt die Löslichkeit des aromatischen Kohlenwasserstoffes V. Bei diesen Reduktionen entstehen als Nebenprodukte oft zusätzlich isomere bicyclische Cyclohexadien-Verbindungen der Formel

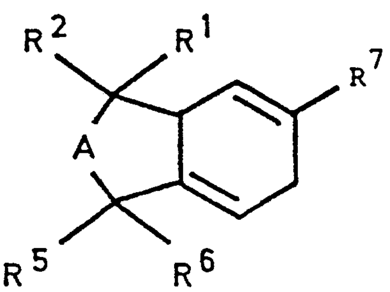

VI

sowie bicyclische Cyclohexen-Derivate der Formel

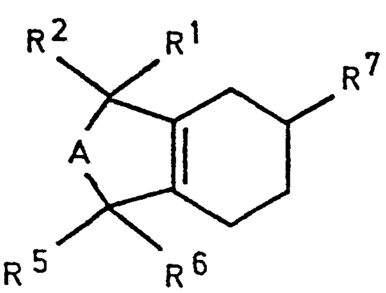

VII

worin A und die Reste $R^1$ bis $R^7$ obige Bedeutung besitzen. Diese Cyclohexene werden bei der erfindungsgemässen Epoxidierungsstufe praktisch nicht umgesetzt und können destillativ von den Epoxiden I abgetrennt werden.

Die Verbindungen I weisen besondere organoleptische Eigenschaften auf, auf Grund derer sie sich vorzüglich als Riech- und/oder Geschmackstoffe eignen.

Die Verbindungen der Formel I zeichnen sich durch kräftige, diffusive und sehr natürlich-warme Kopfnoten in Richtung Moschus, mit fruchtigen und holzigen Geruchsaspekten aus. Daneben ist ein pudrig-blumiger Unterton erwähnenswert. Es handelt sich somit bei I u.a. um eine neuartige Gruppe von Moschus-Riechstoffen, deren bicyclisches Kohlenstoffgerüst statt der bisher in diesen Systemen bekannten Acetylgruppe eine 1,2-Epoxy-Gruppierung (Oxiran) als charakteristisches Merkmal trägt.

Die Erfindung betrifft demgemäss auch die Verwendung der Verbindungen I als Riech- und/oder Geschmackstoffe.

Die Verbindungen der Formel I eignen sich aufgrund ihrer natürlichen Geruchsnoten insbesondere zur Modifizierung von bekannten Kompositionen.

Die Verbindungen I verbinden sich mit zahlreichen bekannten Riechstoffingredientien natürlichen oder synthetischen Ursprungs, wobei die Palette der natürlichen Rohstoffe sowohl leicht- als auch mittel- und schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl Paraguay, Wermutöl
- Alkohole, wie Geraniol, Linalool, Nerol, Phenyläthylalkohol, Rhodinol, Zimtalkohol - Aldehyde, wie Citral, Helional®, α-Hexylzimtaldehyd, Hydroxycitronellal, Lilial® (p-tert. Butyl-α-methyldihydrozimtaldehyd), Methylnonylacetaldehyd
- Ketone, wie Allyljonon, α-Jonon, β-Jonon, Methyljonon
- Ester, wie Allyl-phenoxyacetat, Benzyl-salicylat, Cinnamylpropionat, Dimethylbenzylcarbinyl-butyrat, Aethyl-acetoacetat, Linalylacetat, Methyl-dihydrojasmonat, Styrallylacetat, Vetiverylacetat
- Lactone, wie γ-Undecalacton
- verschiedene in der Parfümerie oft benützte Komponenten, wie Ketonmoschus, Indol, p-Menthan-8-thiol-3-on, Methyleugenol.

Bemerkenswert ist ferner die Art und Weise, wie die Verbindungen I die Geruchsnoten bekannter Kompositionen abrunden und harmonisieren, ohne aber zu dominieren. So unterstreichen sie z.B. in Parfümbasen mit Tee- und Grün-Charakter die weiche und blumige Note, und in Rosenbasen wird der gesuchte Charakter der schweren und süssen bulgarischen Rose unterstrichen.

In Fruchtbasen können die Verbindungen I mit Erfolg zum Erzielen eines samtig-weichen, natürlich-süssen und abrundenden Effekts in Richtung Pfirsich und Aprikose eingesetzt werden.

Die Verbindungen der Formel I (bzw. deren Gemische) lassen sich in weiten Grenzen einsetzen, die beispielsweise von 0,1 (Detergentien) bis 30% (alkoholische Lösungen) in Kompositionen reichen können ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0 5 und 25%. Die mit I hergestellten Kompositionen lassen sich für alle Arten von parfümierten Verbrauchsgütern einsetzen (Eaux de Cologne, Eaux de Toilette, Extraits Lotionen, Crémes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben angeführten bekannten Riechstoffe nach (dem Parfümeur bekannter) Art und Weise verwendet werden, wie z.B. aus W.A. Poucher, Perfumes, Cosmetics, Soaps 2, 7. Auflage, Chapman und Hall, London 1974 hervorgehend.

Die neuen Verbindungen der Formel I sind ebenfalls vorzüglich geeignet zur Verwendung in Fruchtaromen verschiedenster Art, insbesondere aber auch zur Aromatisierung von Tabak.

Als Geschmackstoffe können die Verbindungen I beispielsweise zur Erzeugung bzw. Verbesserung, Verstärkung Steigerung oder Modifizierung von Fruchtaromen verschiedenster Art, z.B. Brombeer- oder Aprikosenaromen verwendet werden. Als Anwendungsgebiet dieser Aromen kommen beispielsweise Nahrungsmittel (Joghurt, Süsswaren etc.), Genussmittel (Tee, Tabak etc.) und Getränke (Limonade etc.) in Frage.

Die ausgeprägten geschmacklichen Qualitäten der Verbindungen I ermöglichen die Verwendung als Aromastoffe in geringen Konzentrationen. Eine geeignete Dosierung umfasst beispielsweise den Bereich von 0,01 ppm - 100 ppm vorzugsweise den Bereich von 0,01 ppm - 20 ppm, im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50 - 500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw.

in essbaren Materialien verteilen lassen. Sie enthalten beispielsweise etwa 0,1 - 10, insbesondere 0,5 - 3 Gew.% an Verbindungen I. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung enthalten oder können der einschlägigen Literatur entnommen werden, siehe z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio 1975.

Für die Herstellung der üblichen Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser Aethanol, Propylenglykol, Glycerin.

**Beispiel 1**

**Methode A**

0,544 g eines Kohlenwasserstoffgemisches, enthaltend rund 37,5% (nach GC) 1,1,4 4 6-Pentamethyl-1 2 3-4,5,8-hexahydronaphthalin (IIa) werden in 8 ml Aceton gelöst und mit einer Lösung von 0,25 g Natriumdihydrogenphosphatmonohydrat in 1 ml Wasser versetzt. Dazu wird eine Lösung von 0,132 g 65%igem Calciumhypochlorit in 1 ml Wasser und nach 30-minutigem Rühren nochmals 0,25 g Natriumdihydrogenphosphat-monohydrat und 0,132 g 65%iges Calciumhypochlorit in 1 ml Wasser gegeben. Nach 1 1/2 Stunden Rühren wird in Dichlormethan aufgenommen und mit Kochsalzlösung gewaschen. Nach Entfernen des Lösungsmittels erhält man 0,54 g Rohprodukt. Nach Abdestillieren der nicht umgesetzten Kohlenwasserstoffe verbleiben 0,082 g (32% Ausbeute bezogen auf IIa) 7-Chloro-6-hydroxy-1,1,4,4,6-pentamethyl-1,2,3,4,5,6,7,8-octahydronaphthalin, das, aus Hexan umkristallisiert, bei 122-124° C schmilzt.

0,3 g obigen Chlorhydrins werden mit 6 ml einer 1 n methanolischen Kaliumhydroxidlösung versetzt und 30 Minuten bei Raumtemperatur gerührt. Darauf wird in Hexan aufgenommen und mit Wasser neutral gewaschen. Nach dem Einengen des Lösungsmittels verbleiben 0,24 g Oel, das bei 85° C 0,066 mbar (0,05 Torr) im Kugelrohr destilliert wird: man erhält 0,19 g (75% Ausbeute) 6,7-Epoxy-1,1,4,4,6-pentamethyl-1,2,3,4,5,6,7,8-octahydronaphthalin (Ia).

$$n_D^{20} = 1,4973$$

$^1$H-NMR (400 mHz, $CDCl_3$): δ (ppm) 0,95 (s, 6H); 0,98 (s, 6H); 1,37 (s, 3H); 3,08 (m, IH)
MS (m/e): 220 (M+), 205, 187, 177, 161, 147, 121, 105

$$n_D^{20} = 1,4960$$

**Methode B**

0,742 g eines Kohlenwasserstoffgemisches, enthaltend rund 27,5% (nach GC) 1,1,4,4,6-Pentamethyl-1,2,3,4,5,8-hexahydronaphthalin (IIa) und 0,01 g Natriumacetat werden mit 3 ml Dichlormethan versetzt. Bei 10° C wird eine Lösung von 0,215 ml 40%iger Peressigsäure und 0,01 g Natriumacetat in 0,5 ml Dichlormethan zugegeben und bei Raumtemperatur 2 1/2 Stunden gerührt. Darauf werden 0,2 ml einer gesättigten Natriumhydrogensulfitlösung und 0,8 ml Wasser zugefügt und 2 1/2 Stunden gerührt. Man nimmt in Dichlormethan auf und wäscht mit Natriumhydrogencarbonatlösung und Wasser neutral. Nach dem Trocknen und Absaugen des Lösungsmittels verbleiben 0,65 g eines Oeles, welches mittels präparativer Dickschichtchromatographie (Merck Kieselgelplatten, Fliessmittel (2x): Dichlormethan) in seine Komponenten getrennt wird. Aus der Schicht mit Rf = 0,58 isoliert man 0,018 g 4a,8a-Epoxy-1,1,4,4,6-pentamethyl-1,2,3,4,4a,5,8,8a-octahydronaphthalin (IVa).

$^1$H-NMR (400 mHz, $CDCl_3$): δ (ppm) 1,01 (s, 3H); 1,02 (s 3H); 1,03 (s, 3H); 1,05 (s, 3H); 1,63 (s, 3H); 5,16 (m, 1H)
MS (m/e): 220 (M+), 205, 187, 177, 151, 121, 109, 107 Geruch: beeren- und minzartig; schwach

Die Schicht mit Rf = 0,47 liefert 0,068 g 6 7-Epoxy-1,1,4,4,6-pentamethyl-1,2,3,4,5,6,7,8-octahydronaphthalin (Ia).

**Methode C**

200,7 g eines Kohlenwasserstoffgemisches. enthaltend rund 62,6% (nach GC) 1,1,4,4,6-Pentamethyl-1,2,3-4,5,8-hexahydronaphthalin (IIa), werden in 590 ml 1,2-Di-chloräthan gelöst und bei 20°C mit 1,62 g Molybdänhexacarbonyl sowie 0,62 g wasserfreiem Dinatriumhydrogenphosphat versetzt. Man erhitzt auf 75-80°C Innentemperatur und tropft ohne weitere Wärmezufuhr innerhalb von 20 Minuten 324 ml einer ca. 2,85 molaren wasserfreien Lösung von tert. Butylhydroperoxid in 1,2-Dichloräthan zu. Man hält während 3 1/2 Stunden bei Rückflusstemperatur, kühlt danach auf 5°C ab, fügt innerhalb von 20 Minuten 210 ml 20%ige Natriumsulfitlösung zu und rührt kräftig während weiteren 2 1/2 Stunden. Man nimmt in Dichlormethan auf, wäscht mit Wasser neutral und erhält nach dem Trocknen und Einengen des Lösungsmittels 208 6 g eines gelben Oeles. Dieses wird am Hochvakuum über eine Widmer-Kolonne destilliert. Die bei 72-80°C 0,026 mbar (0,02 Torr) siedende Fraktion (88,2 g; 65% Ausbeute bez. IIa) stellt das 6,7-Epoxy-1,1,4,4,6-pentamethyl-1,2,3,4,5,6,7,8-octahydronaphthalin (Ia) dar.

Geruch: moschusartig, an Ambrettesamen erinnernd, fruchtig.

D. Herstellung des Ausgangsmaterials

Das Kohlenwasserstoffgemisch wird wie folgt erhalten:

In einem geeigneten Reaktionsgefäss werden 160 ml Methylamin bei -15°C vorgelegt und mit 40 g 1 1,4,4,6-Pentamethyl-1,2,3,4-tetrahydronaphthalin (Va) sowie mit 18 g Aethanol versetzt. Zu dieser Lösung gibt man bei -15°C 2,7 g Lithium portionenweise hinzu. Nach dem Verschwinden des Lithiums (ca. 10 Minuten) gibt man noch 9 g Aethanol und anschliessend 1,4 g Lithium hinzu. Nach weiteren 15 Minuten hat das Lithium vollkommen ausreagiert und das Methylamin wird abdestilliert. Der Rückstand wird in Hexan aufgenommen, mit Wasser neutral gewaschen und getrocknet. Nach dem Absaugen des Lösungsmittels verbleiben 40 g Rohprodukt. Dieses enthält folgende Substanzen (Flächenprozente nach GC in Klammern; präparativ-gaschromatographische Isolierung): IIa:(55%) $^1$H-NMR (400 mHz, $CDCl_3$): δ (ppm) 0,99 (s, 6H);
1,01 (s, 6H); 1,68 (m, 3H); 5,42 (m, 1H)
MS (m/e): 204 (M+), 189, 133, 119, 105
VIa:(7%) $^1$H-NMR (400 mHz $CDCl_3$): δ (ppm) 0,63 (s, 3H);
0,99 (s, 3H); 1,03 (s, 3H); 1,07 (s, 3H); 1,69
(s, 3H); 5,43 (m, 1H); 5,50 (t, 1H)
MS (m/e): 204 (M+), 189, 135, 133, 119, 105
VIIa:(30%) $^1$H-NMR (400 mHz, $CDCl_3$): δ (ppm) 0,93 (d, 3H);
0,93 (s, 6H); 0,96 (s, 6H);
MS (m/e): 206 (M+), 191, 162, 150, 135, 121, 109

**Beispiel 2**

108,5 g eines Kohlenwasserstoffgemisches, enthaltend rund 57,6% (nach GC) 1,1,3,4,4,6-Hexamethyl-1,2,3,4,5,8-hexahydronaphthalin (IIb), werden in 236 ml 1,2-Dichloräthan gelöst und mit 0,755 g Molybdänhexacarbonyl, 0,286 g Dinatriumhydrogenphosphat sowie 150,5 ml 2,85 molarer tert. Butylhydroperoxidlösung - gemäss der in Beispiel 1 beschriebenen Methode C - behandelt. Nach Aufarbeitung und Hochvakuumdestillation erhält man eine bei 68-79°C 0,026 mbar (0,02 Torr) siedende Fraktion (47,2 g; 70,5% Ausbeute bez. IIb); diese besteht aus einem Diastereomerengemisch von 6,7-Epoxy-1,1,3,4,4,6-hexamethyl-1,2,3,4,5,6,7,-8-octahydronaphthalin und 5,6-Epoxy-1,1,3,4,4,6-hexamethyl-1,2,3,4,4a,5,6,7-octahydronaphthalin im Verhältnis von ca. 92:8 und weist folgende Daten auf:

$$n_D^{20} = 1,4986$$

$^1$H-NMR (400 mHz, $CDCl_3$): δ (ppm) 0,77 + 0,80 (2s, 3H); 0,85 + 0,86 (2d, 3H); 0,92 + 0,94 (2s, 3H); 0,97 (s, 3H); 0,99 (s, 3H); 1,37 + 1,375 (2s, 3H); 3,08 (m, ca. 1H); 3,14 (m, ca. 0,1H); 5,23 (m, ca. 0,1H)
MS (m/e): 234 (M+) 219, 201, 191, 175, 161, 149, 135, 121, 107
Geruch: moschus- und birnenartig.

Aus dem eingesetzten Kohlenwasserstoffgemisch kann die Komponente (IIb) durch mehrmalige Umkristallisation aus Hexan rein isoliert und anschliessend gemäss Methode C des Beispiels 1 epoxidiert werden. Das so erhaltene 6,7-Epoxid (Diastereomerengemisch ca. 1:1 nach NMR) weist folgende Daten auf:
$^1$H-NMR (400 mHz, $CDCl_3$): δ (ppm) 0,77 + 0,80 (2s, 3H); 0,85 + 0,86 (2d, 3H); 0,92 + 0 94 (2s, 3H); 0,97 (s, 3H); 0,99 (s 3H); 1,37 + 1,375 (2s, 3H); 3,08 (m, 1H)

MS (m/e): 234 (M+), 219, 201, 191, 175, 161, 149, 135, 121, 107
Geruch: moschusartig.

Aus dem eingesetzten Kohlenwasserstoffgemisch kann ferner die Komponente (VIb) präparativ gaschromatographisch abgetrennt und anschliessend gemäss Methode C (Beispiel 1) epoxidiert werden. Das so erhaltene 5,6-Epoxid weist folgende Daten auf:

$^1$H-NMR (400 mHz, $CDCl_3$): δ (ppm) 0,57 (s, 3H); 0,79 (d, 3H); 1,02 (s, 3H); 1,04 (s, 3H); 1,16 (s, 3H); 1,35 (s, 3H); 3,14 (m 1H); 5,23 (m, 1H)

MS (m/e): 234 (M+), 216, 201, 191, 159, 145, 135, 121 109 Geruch: fruchtig, leicht holzig, leicht moschusartig, schwach.

**Herstellung des Ausgangsmaterials**

Das obige Kohlenwasserstoffgemisch wird analog zu der in Beispiel 1 beschriebenen Herstellung des dortigen Ausgangsmaterials wie folgt synthetisiert: 172,8 g 1,1,3,-4,4,6-Hexamethyl-1,2,3,4-tetrahydronaphthalin werden in 1,15 l Methylamin sowie 80 ml Tetrahydrofuran bei -15°C gelöst und mit insgesamt 228 ml Aethanol sowie 27 g Lithium (in je 4 Portionen) umgesetzt. Nach dem Aufarbeiten verbleiben 179 g Rohprodukt, welches sich aus folgenden Substanzen zusammensetzt (Flächenprozente nach GC in Klammer; präparativ-gaschromatographische Isolierung):

Isomer IIb: $^1$H-NMR (400 mHz, $CDCl_3$): δ (ppm) 0,83 (s,
(61,5%) 3H); 0,88 (d, 3H); 0,96 (s, 3H); 1,00 (s 3H); 1,02 (s, 3H); 1,69 (s, 3H); 5,42 (m, 1H)
MS (m/e): 218 (M+), 203, 175, 159, 147 133, 119, 105 Smp: 46-48°C
Isomer VIb: $^1$H-NMR (400 mHz, $CDCl_3$): δ (ppm) 0,47 (s
(4,9%) 3H); 0,78 (d, 3H); 0,99 (s, 3H); 1,05 (s 3H); 1,06 (s, 3H); 1,70 (s, 3H); 5,49 (m, 2H)
6,7-Dihydro- $^1$H-NMR (360 mHz, $CDCl_3$): δ (ppm) 0 77 (d,
IIb (=VIIb) 3H); 0,85 (2d, 3H); 0,88 bis 1,0 (7s, 12H)
(32,5%) MS (m/e): 220 (M+), 205, 191, 177, 163, 149, 135, 121, 109

**Beispiel 3**

44,5 g eines Kohlenwasserstoffgemisches, enthaltend (nach GC) rund 85,4% 1,1,3,3,5-Pentamethyl-4,7-dihydroindan (IIc), werden in 200 ml 1,2-Dichloräthan gelöst und mit 0,53 g Molybdänhexacarbonyl, 0,2 g Dinatriumhydrogenphosphat sowie 140 ml 2,85-molarer tert. Butylhydroperoxidlösung - gemäss der in Beispiel 1 beschriebenen Methode C - behandelt. Nach der Aufarbeitung und Hochvakuumdestillation erhält man 23,6 g (entsprechend 57,2% Ausbeute bez. IIc), 5,6-Epoxy-1,1,3,3,5-pentamethyl-4,5,6,7-tetrahydroindan, mit folgenden Daten:

$$n_D^{20} = 1,4790$$

$^1$H-NMR (400 mHz, $CDCl_3$): δ (ppm) 1,00 (s, 12H); 1,41 (s, 3H); 3,13 (m, IH)

MS (m/e): 206 (M+), 191, 173, 163, 147, 133, 121, 107, 105 Geruch: moschus- und trockenfrüchte-artig, zedernholz-artig, fruchtig.

**Herstellung des Ausgangsmaterials**

Das obige Kohlenwasserstoffgemisch wird analog der in Beispiel 1 für das dortige Ausgangsmaterial beschriebenen Herstellung wie folgt synthetisiert: 56,4 g 1,1,3,3,5-Pentamethyl-indan werden bei -15°C in 240 ml Methylamin gelöst und mit total 36,3 ml Aethanol sowie 6,08 g Lithium (je 2 Portionen) umgesetzt. Nach dem Aufarbeiten verbleiben 56,3 g Rohprodukt, welches sich aus folgenden Substanzen zusammensetzt (Flächenprozente nach GC in Klammer; präparativ-gaschromatographisch isoliert):

IIc: $^1$H-NMR (360 mHz, $CDCl_3$): δ (ppm) 1,03 (s, 6H);
(85,4%) 1,05 (s, 6H); 1,74 (s, 3H); 5,49 (m, 1H) MS (m/e): 190 (M+), 175, 159, 145, 133, 119 105
5,6-Dihydro- MS (m/e): 192 (M+), 177, 149, 135, 121, 107 IIc (=VIIc):
(11,6%)

**Beispiel 4**

21,2 g eines Kohlenwasserstoffgemisches, enthaltend (nach GC) rund 51,5% 1,1,2,3,3,5-Hexamethyl-4,7-dihydroindan (IId) und 25,7% 3-Aethyl-1,1,3,5-tetramethyl-4,7-dihydroindan(IIe), werden in 80 ml 1,2-Dichloräthan gelöst und mit 0,212 g Molybdänhexacarbonyl, 0,08 g Dinatriumhydrogenphosphat sowie 56 ml 2,85 molarer tert. Butylhydroperoxidlösung gemäss der in Beispiel 1 beschriebenen Methode C behandelt. Nach der Aufarbeitung und Hochvakuumdestillation erhält man eine bei 71-75°C 0,026 mbar (0,02 Torr) siedende Fraktion (10,25 g; 58,2% Ausbeute bez. IId + IIe). Diese enthält gemäss Kapillar-GC drei isomere Epoxide im Verhältnis: 43%:33%:24%. Dieses Epoxidgemisch weist folgende Daten auf:

$$n_D^{20} = 1,4870$$

MS (m/e): 220 (M+), 205, 191, 161, 147, 135, 121, 105 Geruch: moschusartig, holzig.
Die im Kapillar-GC ersichtlichen drei Peaks werden anschliessend präparativ gaschromatographisch getrennt und die so erhaltenen reinen Substanzen analysiert. Beim Hauptprodukt (Peak 1) handelt es sich um eines der diastereomeren 5,6-Epoxy-1,1,2,3,3,5-hexamethyl-4,5,6,7-tetrahydroindane mit folgenden Daten:
$^1$H-NMR (400 mHz, $CDCl_3$): δ (ppm) 0,82 (2s, 6H); 0,85 (d, 3H); 0,93 (2s, 6H); 1,42 (s, 3H); 3,15 (m, 1H)
MS (m/e): 220 (M+), 205, 191, 177, 161, 147, 135, 121, 118, 105
Geruch: moschus- und trockenfrüchte-artig, leicht holzig.
Bei Peak 2 handelt es sich um das zweite diastereomere 5,6-Epoxy-1,1,2,3,3,5-hexamethyl-4,5,6,7-tetrahydroindan mit folgenden Daten:
$^1$H-NMR (400 mHz, $CDCl_3$): δ (ppm) 0,79 (s, 6H); 0,86 (d, 3H); 0 92 (2s, 6H); 1,41 (s, 3H); 3,12 (m, 1H)
MS (m/e): 220 (M+), 205, 191, 177, 161, 147, 135, 121 107, 105
Geruch: holzig, schwach moschusartig.
Bei Peak 3 handelt es sich um das nicht aufgetrennte Diastereomerenpaar (ca. 1:1 nach NMR) von 5,6-Epoxy-3-äthyl-1,1,3,5-tetramethyl-4,5,6,7-tetrahydroindan mit folgenden Daten:
$^1$H-NMR (400 mHz, $CDCl_3$): δ (ppm) 0,73 + 0,76 (2t, 3H); 0,97 bis 1,02 (5s, 9H); 1,40 (s 3H); 3,12 (m, 1H)
MS (m/e): 220 (M+), 205, 191, 173, 161, 147, 133, 121 107, 105
Geruch: Trockenfrüchte-artig, schwach moschus-artig.

**Herstellung des Ausgangsmaterials**

Das Kohlenwasserstoffgemisch dieses Beispiels wird analog der in Beispiel 1 für das dortige Ausgangsmaterial beschriebenen Herstellung wie folgt synthetisiert: 223 g eines Gemisches, bestehend aus rund 70% 1,1,2,3,3,5-Hexa-methylindan sowie rund 30% 3-Aethyl-1,1,3,5-tetramethylindan werden bei -15°C in 700 ml Methylamin gelöst und mit total 151,8 g Aethanol sowie 22,9 g Lithium (in je 2 Portionen) umgesetzt. Nach dem Aufarbeiten verbleiben 245 g Rohprodukt, welche sich aus folgenden Substanzen zusammensetzen (Flächenprozente nach GC in Klammern; präparativgaschromatisch isoliert):
Isomer IId: $^1$H-NMR (400 mHz, $CDCl_3$): δ (ppm) 0,84 (s
(63,0%) 3H); 0,86 (s, 3H); 0,89 (d, 3H); 0,95 (s, 3H); 0,97 (s, 3H); 1,73 (s, 3H); 5,48 (m,1H) MS (m/e): 204 (M+), 189, 147, 133, 119, 107
Isomer IIe: $^1$H-NMR (400 mHz, $CDCl_3$): δ (ppm) 0,78 (t (20,2%) 3H); 1,01 (s, 3H); 1,02 (s, 3H); 1,04 (s, 3H); 1,73 (s, 3H); 5,48 (m, 1H) MS (m/e): 204 (M+), 189, 175, 133, 119, 105
5,6-Dihydro- MS (m/e) 206 (M+), 191, 149, 135, 121, 107 IId (=VIId): (9,7%)
5,6-Dihydro- MS (m/e) 206 (M+), 191, 177, 149, 135, 121, IIe (=VIIe): 107 (3,5%)

**Beispiel 5**

35,8 g eines Kohlenwasserstoffgemisches, enthaltend (nach GC) rund 64,3% 1-Isopropyl-2,3,3,5-tetra-methyl-4,7-dihydroindan (IIf), werden in 105 ml 1,2-Dichlor-äthan gelöst und mit 0,29 g Molybdänhexacarbonyl 0,11 g Dinatriumhydrogenphosphat sowie 57,2 ml 2,85 molarer tert. Butylhydroperoxidlösung gemäss der in Beispiel 1 beschriebenen Methode C behandelt- Nach der Aufarbeitung und Hochvakuumdestillation erhält man eine bei 64-71°C 0,066 mbar (0,05 Torr) siedende Fraktion (13,9 g = 54% Ausbeute bezgl. IIf) bestehend aus 5,6-Epoxy-1-isopropyl-2,3,3,5-tetramethyl-4,5,6,7-tetrahydroindan, mit folgenden Daten:

$$n_D^{20} = 1,4899$$

$^1$H-NMR (400 mHz, $CDCl_3$): δ (ppm) 0,74 (d, 3H); 0,79 + 0,83 (2d, 3H); 0,92 bis 0,94 (m, 9H); 1,39 + 1,40 (2s, 3H); 3,08 + 3 09 (2m, 1H)
MS (m/e): 234 (M+), 219, 205, 191, 173, 159, 147, 133, 121, 105
Geruch: Moschus-artig, fruchtig.

**Herstellung des Ausgangsmaterials**

Das Kohlenwasserstoffgemisch dieses Beispiels wird analog der in Beispiel 1 für das Ausgangsmaterial beschriebenen Herstellung wie folgt synthetisiert: 37,5 g 1-Isopropyl-2,3,3,5-tetramethyl-indan (Vf) werden bei -15°C in 140 ml Methylamin gelöst und mit total 34,5 ml Aethanol sowie 4,08 g Lithium (in je 3 Portionen) umgesetzt. Nach dem Aufarbeiten verbleiben 32,7 g Rohprodukt, welche sich aus folgenden Hauptkomponenten zusammensetzen (Flächenprozente nach GC in Klammern; präparativ-gaschromatographisch isoliert):
Isomer IIf: $^1$H-NMR (400 mHz, $CDCl_3$): δ (ppm) 0,79 (s,
(64,3%) 3H); 0,84 (d, 3H); 0,93 (d, 3H); 0,96 (d, 3H); 0,96 (s, 3H); 1,72 (s, 3H); 5,43 (m, 1H)
MS (m/e): 218 (M+), 203, 175, 159, 147, 133, 119, 105
5,6-Dihydro- MS (m/e): 220 (M+), 205, 177, 163, 149 IIf (=VIIf): 135, 121, 107 (14,3%)

**Beispiel 6**

19,1 g eines Kohlenwasserstoffgemisches enthaltend (nach GC) rund 76,9% 1-Isopropyl-2,2,3,3,5-pentamethyl-4,7-dihydroindan (IIg), werden in 61 ml 1,2-Dichlor-äthan gelöst und mit 0,167 g Molybdänhexacarbonyl, 0,064 g Dinatriumhydrogenphosphat sowie 37,3 ml 2,85 molarer tert. Butylhydroperoxidlösung gemäss der in Beispiel 1 beschriebenen Methode C behandelt. Nach der Aufarbeitung und Hochvakuumdestillation erhält man eine bei 70-74°C 0,093 mbar (0,07 Torr) siedende Fraktion (5,8 g; 37% Ausbeute bez. 17a), bestehend aus 5,6-Epoxy-1-isopropyl-2,2,3,3,5-pentamethyl-4,5,6,7-tetrahydroindan, mit folgenden Daten:

$$n_D^{20} = 1,4975$$

$^1$H-NMR (400 mHz, $CDCl_3$) δ (ppm) 0,73 + 0,75 + 0,78 + 0,79 + 0,80 (5s, 9H); 0,88 (2s, 3H); 0,95 (2d, 3H); 1,01 + 1,03 (2d, 3H); 1,39 + 1,41 (2s, 3H); 3,08 (m, 1H)
MS (m/e): 248 (M+) 233, 205, 187, 177, 161, 145, 135, 121, 109, 105
Geruch: schwach moschus-artig.

**Herstellung des Ausgangsmaterials**

Das Kohlenwasserstoffgemisch dieses Beispiels wird analog der in Beispiel 1 für das Ausgangsmaterial beschriebenen Herstellung synthetisiert: 20,0 g 1-Iso-propyl-2,2,3,3,5-pentamethyl-indan werden bei -15°C in 71 ml Methylamin gelöst und mit total 20,2 ml Aethanol sowie 2,4 g Lithium (je 3 Portionen) umgesetzt. Nach dem Aufarbeiten verbleiben 19,1 g Rohprodukt, welches folgende Hauptsubstanzen enthält (Flächenprozente nach GC in Klammern; präparativ-gaschromatographisch isoliert):
Isomer IIg: $^1$H-NMR (400 mHz, $CDCl_3$): δ (ppm) 0,80 + 0 81 +
(76,8%) 0,83 + 0,92 (4s, 12H); 0,96 (d, 3H); 1,03 (d 3H); 1,73 (s, 3H); 5,44 (m, 1H)
MS (m/e): 232 (M+), 217, 189, 175, 159, 147, 133, 119, 105
5,6-Dihydro- MS (m/e): 234 (M+), 219, 191, 177, 163, 149 IIg (=VIIg) 135, 121, 107 (13,2%)

**Beispiel 7**

35 g eines Kohlenwasserstoffgemisches, enthaltend (nach GC) 43% 1,1,4,4-Tetramethyl-1,2,3,4,5,8-hexahydro-naphthalin (IIh) werden in 140 ml 1,2-Dichloräthan gelöst und mit 0 304 g Molybdänhexacarbonyl, 0,1148 g Dinatriumhydrogenphosphat sowie 65 ml 2 85 molarer tert. Butylhydroperoxidlösung gemäss der in Beispiel 1 beschriebenen Methode C - behandelt. Nach Aufarbeitung erhält man 30 g Rohprodukt. 10 g davon werden auf 600 g Kieselgel Merck (0,04-0,063 mm) chromatographiert (Elution: 2-3% Aether/ Hexan). Man erhält 1,9 g 6,7-Epoxy-1,1,4,4-tetramethyl-1,2,3,4,5,6,7,8-octahydro-naphthalin.

$^1$H-NMR ($CDCl_3$ 400 mHz): 0,95 (s, 6H); 0,98 (s, 6H); 1,45 (m, 4H); 2,31 (d, J=18,2H); 2,61 (d, J=18,2H); 3,26 (s, 2H)

MS (m/e) 206 (M+ 49) 191 (76); 173 (21); 147 (100); 133 (23); 107 (21); 91 (10); 60 (2)

Geruch: Moschus-artig, fruchtig, (ähnlich wie Thibetolid).

2 Das für diese Umsetzung benötigte Kohlenwasserstoffgemisch wird analog der in Beispiel 1 für das Ausgangsmaterial beschriebenen Herstellung synthetisiert: 205 g 1,1,4,4-Tetramethyl-1,2,3,4-tetrahydronaphthalin werden in 800 ml Methylamin bei -15°C gelöst und mit zwei Portionen Aethanol (101,2 g und 50,6 g) und Lithium (15,3 g und 7,6 g) umgesetzt. Nach dem Aufarbeiten verbleiben 198 g Rohprodukt, welches sich aus folgenden Hauptkomponenten zusammensetzen (Flächenprozente nach GC; präparativ-gaschromatographisch isoliert):

31% 1,1,4,4-Tetramethyl-1,2,3,4,5,6,7,8-octahydro-naphthalin,

50% 1,1,4,4-Tetramethyl-1,2,3,4,5,8-hexahydro-naphthalin (IIh)

$^1$H-NMR ($CDCl_3$ 400 mHz): 0,99 (s, 12H); 1,5 (s, 4H); 2,64 (d, J=1,8, 4H); 5,72 (s, breit, 2H)

MS: 190 M+, 175, 119, 105, 91

**Beispiel 8**

6,1 g eines Kohlenwasserstoffgemisches, enthaltend (nach GC) 59% 1,1,2,3,3-Pentamethyl-4,7-dihydroindan (IIj) werden in 15 ml 1,2-Dichloräthan gelöst und mit 53 mg Molybdänhexacarbonyl, 20 mg Dinatriumhydrogenphosphat sowie 11,4 ml 2,85 molarer tert. Butylhydroperoxidlösung gemäss Beispiel 1, Methode C behandelt. Nach der Aufarbeitung erhält man 4 g Rohprodukt. Durch Chromatographie über 300 g Kieselgel Merck (0,04-0,063 mm) werden folgende Einzelverbindungen isoliert:

cis- oder trans-5,6-Epoxy-1,1,2,3,3-pentamethyl-4,5,6,7-tetrahydroindan (Isomer A)

$^1$H-NMR ($CDCl_3$ 400 mHz) 0,79 (s, 6H); 0,85 (d, J=7 Hz 3H); 0,92 (s, 6H); 1,47 (q, J=7 Hz, 1H); 2,3 (d, breit J= 16 Hz, 2H); 2,45 (d, breit, J=16 Hz, 2H); 3,3 s, breit, 2H)

MS 206, 191, 177, 145, 135, 121, 105, 91, 65, 41

cis oder trans 5,6-Epoxy-1,1,2,3,3-pentamethyl-4,5,6,7-tetrahydroindan (Isomer B)

$^1$H-NMR ($CDCl_3$ 400 mHz) 0,81 (s, 6H); 0,84 (d, J=7 Hz 3H); 0,92 (s, 6H); 1,54 (q, J= 7 Hz 1H); 2,27 (d, breit, J=15 Hz, 2H); 2,53 (d, breit, J=15 Hz, 2H); 3,33 (s breit, 2H)

MS M+ 206, 191, 173, 147, 135, 121, 107, 105, 91, 87 65 55, 41

Geruch des Gemisches der Verbindungen: Moschus-artig leicht fruchtig, holzig.

Das benötigte Kohlenwasserstoffgemisch wird analog der im Beispiel 1 für das Ausgangsmaterial beschriebenen Herstellung wie folgt synthetisiert: 207 g eines Gemisches von 1,1,2,3,3-Pentamethylindan (75%) und 3-Aethyl-1,1,3-trimethylindan (17%) werden in 700 ml Methylamin bei -15° gelöst und mit zwei Portionen Aethanol (101 g und 50 g) und Lithium (15,3 g und 7,6 g) umgesetzt. Nach dem Aufarbeiten verbleiben 221 g Produkt, welche sich aus folgenden Substanzen zusammensetzen (Flächenprozente nach GC; präparativ-gaschromatographisch isoliert):

59% 1,1,2,3,3-Pentamethyl-4,7-dihydroindan (IIj)

$^1$H-NMR ($CDCl_3$ 400 mHz) 0,84 (s, 6H); 0,88 (d, J=7 Hz, 3H); 0,95 (s, 6H); 1,55 (q, J=7 Hz, 1H); 2,61 (m, 4H); 5,77 (m, 2H)

MS 190 M+, 175, 147, 133, 119, 105, 91, 69, 55, 41

12% 3-Aethyl-1,1,3-trimethyl-4,7-dihydroindan (IIk)

$^1$H-NMR ($CDCl_3$ 400 mHz) 0,77 (t, J=7 Hz, 3H); 1,01 (s, 6H); 1,05 (s, 3H); 1,32 (m, J=7 Hz, 2H); 1,45 (d, J=13 Hz, 1H); 1,7 (d, J=13 Hz, 1H); 2,56 (m, 4H), 5,77 (m, 2H)

MS 190, 175, 161, 145, 133, 119, 105, 91, 79

20% 1,1,2,3,3-Pentamethyl-4,5,6,7-tetrahydroindan (5,6-Dihydro-IIj).

**Beispiel 9**

81 g eines Kohlenwasserstoffgemisches, enthaltend (nach GC) 69% 3-Aethyl-1,1,3-trimethyl-4,7-dihydroindan (IIk) werden in 600 ml 1,2-Dichloräthan gelöst und mit 795 mg Molybdänhexacarbonyl, 300 mg Dinatriumhydrogenphosphat sowie 180 ml 2,85 molarer tert. Butylhydroperoxidlösung gemäss Beispiel 1, Methode C behandelt. Nach der Aufarbeitung erhält man 90 g Rohprodukt, das (nach GC) 61% eines Gemisches von cis- und trans-5,6-Epoxy-3-äthyl-1,1,3-tri-methyl-4,5,6,7-tetrahydroindan enthält.

Spektrale Daten des (cis- und trans-)Gemisches:

$^1$H-NMR: 0,74 (3H, t, J=8 Hz); 0,76 (3H, t, J=8 Hz); 0,795 (3H, s); 0,985 (3H, s); 0,985 (3H, s); 0,99 (3H, s); 1,01 (3H, s); 1,015 (3H s); 3,3 (2H m); 3,3 (2H, m)

MS: 206 M+, 191, 177, 169, 147, 133

Geruch: Damasconartig, fruchtig, holzig, schwach moschusartig.

Das benötigte Kohlenwasserstoffgemisch wird analog der im Beispiel 1 für das Ausgangsmaterial beschriebenen Herstellung wie folgt synthetisiert: 86 g 3-Aethyl-1,1,3-trimethylindan werden in 600 ml

Methylamin gelöst und bei -15°C mit je zwei Portionen Aethanol (44 g und 22 g) und Lithium (6,3 g und 3,15 g) umgesetzt. Nach dem Aufarbeiten verbleiben 83 g Produkt, welches sich aus folgenden Substanzen zusammensetzen (Flächenprozente nach GC; präparativ-gaschromatographisch isoliert):

69% 3-Aethyl-1,1,3-trimethyl-4,7-dihydroindan (IIk)

$^1$H-NMR ($CDCl_3$) 400 mHz): 0,775 (3H, t, J=8 Hz); 1,017 (6H, s); 1,05 (3H, s); 2,56 (4H, m); 5,775 (2H, m)

MS: 190 M+, 175, 161, 145, 133, 131, 119, 105

16% 3-Aethyl-1 1,3-trimethyl-4,5,6,7-tetrahydroindan.

**Beispiel 10**

8 g eines Kohlenwasserstoffgemisches, enthaltend (nach GC) 54% 1,1,3,4,4-Pentamethyl-1,2,3,4,5,8-hexahydronaphthalin (II 1) werden in 40 ml Dichloräthan gelöst und mit 57 mg Molybdänhexacarbonyl, 21,6 mg Dinatriumhydrogenphosphat sowie 12,31 ml 2,85 molarer tert. Butylhydroperoxidlösung gemäss Beispiel 1, Methode C behandelt. Nach der Aufarbeitung erhält man 9,7 g Rohprodukt. Durch Chromatographie erhält man ein Gemisch von cis- und trans-6,7-Epoxy-1,1,3,4,4-pentamethyl-1,2,3,4,5,6,7,8-octa-hydronaphthalin. Spektrale Daten des cis-/trans-Gemisches:

$^1$H-NMR: ($CDCl_3$, 400 mHz): 0,775 (3H, s); 0,795 (3H, s); 0,850 (3H, d, J=7); 0,860 (3H d, J=7); 0,930 (3H, s); 0,945 (3H, s); 0,972 (3H, s); 0,987 (3H, s); 0,99 (3H, s); 3,27 (2H, m); 3,27 (2H, m)

MS: 220 M+, 205, 187, 161, 147, 135, 121, 107, 91, 79, 69

Geruch: sehr stark moschus-artig, pudrig, suss.

Das benötigte Kohlenwasserstoffgemisch wird analog der im Beispiel 1 für das Ausgangsmaterial beschriebenen Herstellung wie folgt synthetisiert: 8,6 g 1,1,3,4,4-Pentamethyl-1,2,3,4-tetrahydronaphthalin werden bei -15°C in 150 ml Methylamin gelöst und mit je zwei Portionen Aethanol (3,92 g und 1,95 g) und Lithium (570 mg und 293 mg) umgesetzt. Nach dem Aufarbeiten verbleiben 8,4 g Rohprodukt, welche sich aus folgenden Substanzen zusammensetzen (Flächenprozente, präparative Gaschromatographie).

54% 1,1,3,4,4-Pentamethyl-1,2,3,4,5,8-hexahydro-naphthalin

$^1$H-NMR ($CDCl_3$ 400 mHz): 0,802 (3H, s); 0,88 (3H, d, J=7 Hz); 0,967 (3H, s); 0,98 (3H, s); 1,02 (3H s); 5,735 (2H, m)

MS: 204 M+ 189, 167, 145, 135, 119, 105, 91, 83

43% 1,1,3,4,4-Pentamethyl-1,2,3,4,5,6,7 8-octahydro-naphthalin

$^1$H-NMR ($CDCl_3$ 400 mHz): 0,78 (3H, s); 0,85 (3H, d J=8 Hz); 0,915 (3H, s); 0,925 (3H, s); 0,98 (3H, s)

MS: 206, 191, 175, 161, 149, 135, 123, 109

**Beispiel 11**

137 g eines Kohlenwasserstoffgemisches, enthaltend (nach GC) 40% eines Gemisches von 1-Aethyl-1,4,4,6-tetra-methyl-1,2,3,4,5,8-hexahydronaphthalin (IIm) und 1-Aethyl-1,4,4,7-tetramethyl-1,2,3,4,5,8-hexahydronaphthalin (IIn) werden in 400 ml Dichloräthan gelöst und mit 1 g Molybdänhexacarbonyl, 0,35 g Dinatriumhydrogenphosphat sowie 175 ml 2,85 molarer tert. Butylhydroperoxidlösung gemäss Beispiel 1, Methode C behandelt. Nach der Aufarbeitung erhält man 152 g Rohprodukt. Durch Chromatographie erhält man ein Gemisch von 6,7-Epoxy-1-äthyl-1,4,4,6-tetra-methyl-1,2,3,4,5,6,7,8-octahydronaphthalin (cis und trans) und 6,7-Epoxy-1-äthyl-1,4,4,7-tetramethyl-1,2,3,4,5,6,7,8-octahydronaphthalin (cis und trans).

$^1$H-NMR ($CDCl_3$ 400 mHz): 0,75 (3H, t, J=7 Hz); 0,940 (3H s); 0,945 (3H, s); 0,985 (3H, s); 0,99 (3H, s); 1,357 (3H, s); 1,37 (3H, s); 3,075 (1H, m)

MS: 234 M+, 205, 191, 177, 161, 141, 135

Geruch: Moschus-artig, balsamisch, an Tannen und Brombeeren erinnernd.

Das benötigte Kohlenwasserstoffgemisch wird analog der im Beispiel 1 für das Ausgangsmaterial beschriebenen Herstellung wie folgt synthetisiert: 237 g eines Gemisches von 1-Aethyl-1,4,4,6-tetramethyl-1,2,3,4-tetrahydronaphth-alin und 1-Aethyl-1,4,4,7-tetramethyl-1,2,3,4-tetrahydro-naphthalin werden in 700 ml Methylamin gelöst und bei -15°C mit je drei Portionen Aethanol (93 g, 47 g, 47 g) und Lithium (15,2 g, 7,6 g, 7,6 g) umgesetzt. Nach dem Aufarbeiten verbleiben 233 g Material, welche sich aus folgenden Substanzen zusammensetzen (Flächenprozente, präparativ-gaschromatographisch isoliert):

40% eines Gemisches von 1-Aethyl-1,4,4,6-tetramethyl-1,2,3,4,5,8-hexahydronaphthalin (IIm) und 1-Aethyl-1,4,4,7-tetramethyl-1,2,3,4,5,8-hexahydronaphthalin (IIn).

$^1$H-NMR ($CDCl_3$ 400 mHz): 0,737 (3H, t, J=7 Hz); 0,747 (3H, t, J=7 Hz); 0,955 (3H, s); 0,975 (3H, s); 0,982 (3H, s); 0,990 (3H, s); 1,002 (3H, s); 1,007 (3H, s); 1,68 (3H, s breit); 1,68 (3H, s, breit); 5,42 (1H, m); 5,42 (1H, m)

MS: M+ 218, 203, 189, 175, 173, 159, 147, 133, 119, 105 91, 77, 69

45% eines Gemisches von cis- und trans-1-Aethyl-1,4,4,6-tetramethyl-1,2,3,4,5,6,7,8-octahydronaphthalin (Dihydro-IIm = VIIm) und 1-Aethyl-1,4,4,7-tetramethyl-1,2,3,4,5,6,7,8-octahydronaphthalin (Dihydro-IIn = VIIn)

$^1$H-NMR ($CDCl_3$ 400 mHz): 0,892 (s); 0,905 (s); 0,925 (s); 0,945 (s); 0,950 (s); 0,950 (s); 0,965 (s)

MS: M+ 220, 205, 191, 177, 149, 135, 121, 109, 105, 95

**Herstellung des Tetrahydronaphthalins**

210 g 2,5-Dimethyl-hept-1-en-5-ol werden in 469 g konzentrierter Salzsäure gelöst. Man erhitzt auf 50°C und tropft 397 g Schwefelsäure innerhalb 30 Minuten unter Rühren zu. Man rührt noch 30 Minuten weiter, dann wird mit Aether verdünnt, mit Wasser neutral gewaschen und eingedampft. Man erhält auf diese Weise 243 g 2,5-Dimethyl-2,5-dichlorheptan.

Eine Lösung von 240 g 2,5-Dichlor-2,5-dimethyl-heptan in 340 ml Toluol wird innerhalb einer Stunde zu einer Suspension von 20,8 g Aluminiumchlorid in 333 g Toluol getropft. Man lässt 50 Minuten rühren, gibt nochmals 15 g Aluminiumchlorid zu, lässt 10 Minuten reagieren, giesst auf Eis und extrahiert mit Hexan. Die organische Phase wird mit Wasser gewaschen, über Natriumsulfat getrocknet und eingedampft. Man erhält 274 g Rohprodukt, das ein Gemisch (91%) folgender Verbindungen darstellt:

1-Aethyl-1,4,4,6-tetramethyl-1,2,3,4-tetrahydro-naphthalin,
1-Aethyl-1,4,4,7-tetramethyl-1,2,3,4-tetrahydro-naphthalin.

Spektrale Daten des Gemisches:

$^1$H-NMR ($CDCl_3$ 400 mHz): 0,767 (3H, t, J=7 Hz); 0,775 (3H, t J=7 Hz); 1,207 (3H s); 1,217 (3H, s); 1,232 (3H, s); 1,242 (3H, s); 1,272 (3H, s); 1,282 (3H, s); 2,290 (3H, s); 2,297 (3H, s); 6,94 (IH, d, J=8 Hz); 6,94 (1H, d, J=8 Hz); 7,02 (1H s breit); 7,105 (1H, s); 7,11 (1H, d J=8 Hz); 7,2 (1H, d, J=8 Hz)

MS: M+ 216, 187, 173, 157, 155, 145, 131, 115, 105, 91

**Beispiel 12**

105 g eines Kohlenwasserstoffgemisches, enthaltend (nach GC) 56% 1-Isopropyl-4,4,7-trimethyl-1,2,3,4,5,8-hexahydronaphthalin (IIo) werden in 400 ml Dichloräthan gelöst und mit 1,1 g Molybdänhexacarbonyl, 0,4 g Dinatriumhydrogenphosphat sowie 228 ml einer 2,8 molaren tert. Butylhydroperoxidlösung gemäss Beispiel 1, Methode C behandelt. Nach der Aufarbeitung erhält man 115 g Rohprodukt. Durch Chromatographie erhält man reines 6,7-Epoxy-1-isopropyl-4,4,7-trimethyl-1,2,3,4,5,6,7,8-octa-hydronaphthalin.

$^1$H-NMR ($CCl_4$ 400 mHz): 0,695 (3H, d, J=7 Hz); 0,920 (3H, s); 0,937 (3H, d, J=5 Hz); 0,99 (3H, s); 1,282 (3H, s); 2,875 (1H, m)

MS: 234 M+, 191, 178, 173, 103, 147, 133, 119, 105, 91, 77, 91, 77

Geruch: Moschus, pfeffrig, würzig.

Das benötigte Kohlenwasserstoffgemisch wird analog der im Beispiel 1 für das dortige Ausgangsmaterials beschriebenen Methode synthetisiert: 190 g 1-Isopropyl-4,4,7-tri-methyl-1,2,3,4-tetrahydronaphthalin, (Reinheit 75%) werden in 650 ml Methylamin gelöst und bei -15°C mit je zwei portionen Aethanol (80 g und 40 g) und Lithium (12,2 g und 6 g) umgesetzt. Nach dem Aufarbeiten verbleiben 180 g, welche sich aus folgenden Hauptkomponenten zusammensetzen (Flächenprozente nach GC; präparativ-gaschromatographisch isoliert):

56% 1-Isopropyl-4,4,7-trimethyl-1,2,3,4,5,8-hexahydro-naphthalin (IIo)

$^1$H-NMR ($CDCl_3$ 400 mHz): 0,71 (3H, d, J=7 Hz); 0,945 (3H, d, J=7 Hz); 0,975 (3H, s); 0,98 (3H, s); 1,665 (3H, s breit); 5,435 (IH, m)

MS: 218 M+, 203, 175, 159, 145, 133, 119 105 91

20% 1-Isopropyl-4,4,7-trimethyl-1,2,3,4,5,6,7,8-octa-hydronaphthalin (Dihydro-IIo = VIIo)

$^1$H-NMR ($CDCl_3$ 400 mHz): 0,67 (3H, d, J=7 Hz); 0,905 (3H, d, J=7 Hz); 0,937 (3H, s); 0,945 (3H, s);

MS: 220, 205, 177, 162, 149, 135, 121, 106, 95

**Herstellung des Tetrahydronaphthalins**

1050 g Schwefelsäure werden vorgelegt. Ein Gemisch von 1,5 1 Toluol und 497 g 2,6-Dimethyl-hept-5-en-2-ol werden während 35 Minuten unter Kühlen (10°C) zugetropft. Es wird 1,5 Stunden bei Raumtemperatur nachgerührt. Der Ansatz wird nun mit Hexan verdünnt, auf Eis gegossen, neutralgewaschen, getrocknet und eingedampft. Nach Destillation erhält man 455 g 1-Isopropyl-4,4,7-trimethyl-1,2,3,4-tetrahydro-naphthalin (Reinheit 75%).

In den folgenden Anwendungsbeispielen 13A-13J bezeichnen die jeweiligen Zahlenangaben nach dem Begriff "Epoxid" die Nummer der Synthesebeispiele. Die bevorzugten Verbindungen sind "Epoxid 1", "Epoxid 2" und "Epoxid 8", d.h. die in den vorstehend aufgeführten Synthesebeispielen 1, 2 bzw. 8 genannten neuen Epoxide 6,7-Epoxy-1,1,4,4,6-pentamethyl-1,2,3,4,5,6,7,8-octahydronaphthalin, 6,7-Epoxy-1,1,3,4,4,6-hexamethyl-1,2,3,4,5,6,7,8-octahydro-naphthalin bzw. 5,6-Epoxy-1,1,2,3,3-pentamethyl-4,5,6,7-tetrahydroindan.

**Beispiel 13**

A Parfümerie-Base Richtung Cologne

| | Gewichtsteile |
|---|---|
| Myrascone ® Giv (2-Aethyl-3,6,6-trimethyl-2-cyclohexen-1-carbonsäureäthylester) | 160 |
| Hydroxycitronellal | 120 |
| Madrox ® Giv (1-Methyl-1-methoxy-cyclododecan) | 120 |
| Sandela ® Giv (3-Isocamphyl-(5)-cyclohexanol) | 120 |
| Bergamotteöl | 120 |
| Fichtennadelöl | 60 |
| Keton-Moschus | 60 |
| Givescone ® Giv (2-Aethyl-6,6-dimethyl-2-cyclohexen-1-carbonsäureäthylester) | 60 |
| Petitgrainöl synthetisch | 40 |
| Corps Cassis ® Giv (p-Menthan-8-thiol-3-on) | 10 |
| Baummoos-extrakt | 10 |
| Dipropylenglykol (DPG) | 80 |
| | 960 |

Gibt man zu dieser Cologne-Base 40 Teile des Epoxids 4, so wirkt diese viel krautiger und frischer, ist also gut für Herren-Colognes geeignet.

Ein Zusatz von 40 Teilen des Epoxids 2 andererseits bringt einen sehr schönen Moschus-Charakter in das Cologne.

Durch einen Zusatz von 40 Teilen des Epoxids 1 wirkt dieses viel lebendiger, blumiger; die entstandene Komposition ist für ein Damen-Cologne geeignet.

B Parfümerie-Base Richtung Tee

| | Gewichtsteile |
|---|---|
| Linalylacetat | 200 |
| Linalool | 120 |
| Hydroxycitronellal | 120 |
| Madrox ® Giv | 120 |
| Methyldihydrojasmonat | 80 |
| Patchouliblätteröl | 60 |
| Methyleugenol | 40 |
| Cetonial (Acetanisol, p-Methoxyacetophenon) | 40 |
| Basilikumöl | 20 |
| Bornylacetat | 20 |
| Baummoos absolut | 20 |
| Dipropylenglykol | 130 |
| | 970 |

Gibt man zu dieser aromatisch wirkenden Base 30 Teile des Epoxids 1, so wirkt diese sofort viel krautiger, frischer und wesentlich kräftiger.

Ein Zusatz von 30 Teilen des Epoxids 4 bewirkt eine eher blumige Version der Tee-Base.

C Parfümerie-Base Richtung Melone

| | Gewichtsteile |
|---|---|
| Myraldylacetat | 140 |
| Hexenylsalicylat | 80 |
| Methyl-dihydrojasmonat | 60 |
| Aethyl-acetyl-acetat | 60 |
| Cyclamenaldehyd | 50 |
| Verdylacetat (Dihydro-nor-dicyclopentadienyl-acetat) | 50 |
| Lilial ® Giv (p-tert. Butyl-α-methyl-hydrozimt-aldehyd) | 10 |
| Rhodinol | 10 |
| Eugenol | 5 |
| Maltylisobutyrat 10% DPG | 5 |
| Acetanisol | 5 |
| cis-6-Nonenol 10% DPG | 5 |
| Dipropylenglykol | 510 |
| | 990 |

Gibt man zu dieser fruchtigen Base (mit Melonen-Charakter) 10 Teile des Epoxids 1, so wird diese fruchtiger und süsser, der Melonen-Charakter wird stark abgeschwächt: es entsteht nun vielmehr ein krautig-blumiger Effekt, welcher der Base eine exotisch wirkende Fruchtnote verleiht.

Gibt man hingegen 30 Teile des Epoxids 5 zu, so wirkt die Base fruchtiger, saftiger und natürlicher.

D Parfümerie-Base Richtung Rose

| | Gewichtsteile |
|---|---|
| Phenyläthylalkohol | 460 |
| Dipropylenglykol | 260 |
| Geraniol | 80 |
| Zimtalkoholsubst. synth. | 70 |
| Nerol | 60 |
| Cinnamylpropionat | 50 |
| | 980 |

Gibt man zu dieser Base mit allgemein rosigem Charakter 20 Teile des Epoxids 3, so wird diese sehr schön abgerundet, es entsteht der Eindruck einer viel typischeren Rosen-Note. Die Base wirkt insbesondere viel harmonischer und hat mehr Volumen.

Gibt man andererseits 20 Teile des Epoxids 2 zu, so geht der rosige Charakter fast völlig verloren, es dominiert nun der sehr angenehme Moschus-Charakter; die recht einfach aufgebaute Base wirkt schon wie eine fertige Komposition.

Gibt man 20 Teile des Epoxids 5 zu der Base, so resultiert ein fruchtig-samtiger Effekt Richtung der gesuchten Teerose.

E Fruchtige Parfümerie-Base

| | Gewichtsteile |
|---|---|
| Dipropylenglykol | 710 |
| α-Ionon | 160 |
| Dimethylbenzylcarbinyl butyrat | 100 |
| Cetone V (α-Allyljonon) | 80 |
| Fructone ® IFF (2-Methyl-1,3-dioxolan-2-äthylacetat) | 60 |
| Palmarosaöl | 40 |
| γ-Undecalacton | 30 |
| synthetisches Osmanthusöl-substitut | 10 |
| | 950 |

Gibt man zu dieser fruchtigen Base 50 Teile des Epoxids 3, so tritt eine ausgeprägte Aprikosen-Pfirsich-Note stark in den Vordergrund. Der Zusatz von 50 Teilen Epoxid bewirkt einen Moschus-Effekt, welcher sich sehr gut in diese fruchtige Note einfügt. Auch ein Zusatz von 50 Teilen des Epoxids 4 bewirkt eine Moschus-Note. Durch einen Zusatz von 50 Teilen des Epoxids 1 wird die Base alsbald viel weicher und eignet sich nun insbesondere zur Parfümierung von Kosmetika.

F Parfümerie-Base Richtung Tabak

| | Gewichtsteile |
|---|---|
| o-tert. Butylcyclohexylacetat | 400 |
| Jasminöl synthetisch | 300 |
| Ketonmoschus | 40 |
| Sandela ® | 40 |
| Styrallylacetat | 30 |
| Cumarin | 20 |
| Isobutylchinolin 10% DPG | 10 |
| Lavendelöl | 10 |
| Vetiveröl | 10 |
| Galbanumöl | 10 |
| Vassuraöl | 10 |
| Dipropylenglykol | 40 |
| | 920 |

Hier erbringt ein Zusatz von 80 Teilen des Epoxids 7 auf billigem Weg einen attraktiven Moschus-Effekt.

G Parfümerie-Base Richtung Rose

| | Gewichtsteile |
|---|---|
| Phenyläthylalkohol | 300 |
| Geraniol | 250 |
| Jasmin "lavage" (wässriges Destillat) | 200 |
| Citronellol extra | 100 |
| α-Ionon | 40 |
| C-10-Aldehyd 10% DPG | 5 |
| C-11-Aldehyd 10% DPG | 5 |
| | 900 |

Durch einen Zusatz von 100 Teilen des Epoxids 8 erzielt man hier auf einfache Weise einen attraktiven Moschus-Effekt, welcher der Rose Fülle und Wärme verleiht. Durch Zusetzen von 100 Teilen des Epoxids 7 wird das Citronellol, welches in der ursprünglichen Base zu stark dominiert, in ausserordentlicher Weise veredelt; die Base wirkt nun ausgeglichen.

H Parfümerie-Base mit fruchtigem Charakter

| | Gewichtsteile |
|---|---|
| Aethyl-3-methyl-3-phenyl-glycidat | 50 |
| Aethyl-acetyl-acetat (Acetessigsäureäthylester) | 15 |
| Dimethyl-benzyl-butyrat | 15 |
| Maltylisobutyrat | 10 |
| Benzylacetat | 10 |
| Aethylacetat | 5 |
| Zitronenöl | 5 |
| Dipropylenglykol | 795 |
| | 900 |

Mit 100 Teilen des Epoxids 8 bringt man in diese allgemein fruchtige Baseeinen interessanten Effekt Richtung Steinobst. Durch 100 Teile Epoxid 7 entsteht andererseits auf sehr einfache Weise eine sehr schöne Himbeer-Note.

J Parfümerie-Base Richtung Tulpe

| | Gewichtsteile |
|---|---|
| Phenyläthylalkohol | 100 |
| Myraldylacetat ® ([4-(4-Methyl-3-pentenyl)-3-cyclohexen-1-yl]methylacetat) | 100 |
| Methyl-dihydrojasmonat | 100 |
| Acetal CD (Glycerinacetal von Phenylacetaldehyd) | 100 |
| Hydroxycitronellal | 160 |
| Farnesol | 40 |
| Hexylsalicylat | 30 |
| Terpineol | 30 |
| Cyclamenaldehyd | 20 |
| Linalool | 20 |
| Linalylanthranilat | 10 |
| Amylsalicylat | 10 |
| C-11-Aldehyd 10% DPG | 10 |
| Benzylacetat | 8 |
| Hexenylbenzoat | 8 |
| Hexenylacetat 10% DPG | 8 |
| p-Cresyl-isobutyrat 10% DPG | 6 |
| Indol 10% DPG | 6 |
| Syringaaldehyd | 4 |
| Dimethylacetal-hydratropaldehyd 10% DPG | 30 |
| DPG | 100 |
| | 900 |

Ein Zusatz von 100 Teilen Epoxid 8 verleiht dieser Blumenbase einen Moschus-Charakter in Richtung Aethylenbrassilat.

**Patentansprüche**

1. Verbindungen der allgemeinen Formel

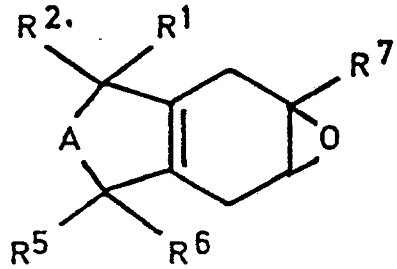

I

worin A für 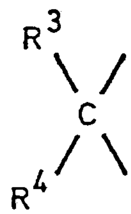 oder 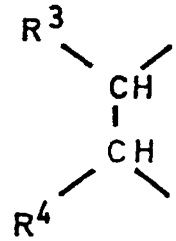 steht und die

Symbole $R^1$, $R^2$, $R^5$ und $R^6$ H, $CH_3$, $C_2H_5$ oder $-CH(CH_3)_2$ darstellen, wobei aber H, $C_2H_5$ oder $-CH(CH_3)_2$ jeweils nur einmal vorhanden ist, und die Symbole $R^3$, $R^4$ und $R^7$ H oder $CH_3$ darstellen.

2. 6,7-Epoxy-1,1,3,4,4,6-hexamethyl-1,2,3,4,5,6,7,8-octahydronaphthalin.

3. 6,7-Epoxy-1,1,4,4,6-pentamethyl-1,2,3,4,5,6,7,8-octahydronaphthalin.

4. 5,6-Epoxy-1,1,2,3,3-pentamethyl-4,5,6,7-tetrahydroindan.

5. Eine Verbindung, ausgewählt aus: 5,6-Epoxy-1,1,3,3,5-pentamethyl-4,5,6,7-tetrahydroindan, 5,6-Epoxy-1,1,2,3,3,5-hexamethyl-4,5,6,7-tetrahydroindan, 5,6-Epoxy-3-äthyl-1,1,3,5-tetramethyl-4,5,6,7-tetrahydro-indan, 5,6-Epoxy-1-isopropyl-2,3,3,5-tetramethyl-4,5,6,7-tetrahydroindan, 5,6-Epoxy-1-isopropyl-2,2,3,3,5-pentamethyl-4,5,6,7-tetrahydroindan, 6,7-Epoxy-1,1,4,4-tetra-methyl-1,2,3,4,5,6,7,8-octahydronaphthalin, 5,6-Epoxy-3-äthyl-1,1,3-trimethyl-4,5,6,7-tetrahydroindan, 6,7-Epoxy-1,1,3,4,4-pentamethyl-1,2,3,4,5,6,7,8-octahydronaphthalin 6,7-Epoxy-1-athyl-1,4,4,6-tetramethyl-1,2,3,4,5,6,7,8-octahydronaphthalin, 6,7-Epoxy-1-isopropyl-4,4,7-trimethyl-1,2,3,4,5,6,7,8-octahydronaphthalin, 6 7-Epoxy-1-äthyl-1,4,4,7-tetramethyl-1,2,3,4,5,6,7,8-octahydronaphthalin.

6. Verbindungen der allgemeinen Formel

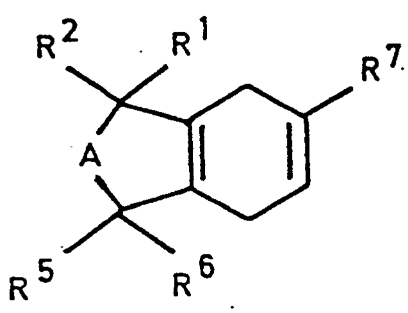

II

worin A für 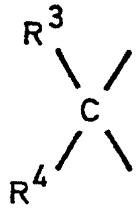 oder 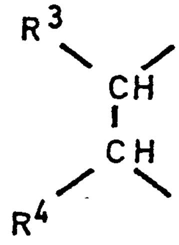 steht und die

Symbole $R^1$, $R^2$, $R^5$ und $R^6$ H, $CH_3$, $C_2H_5$ oder $-CH(CH_3)_2$ darstellen wobei aber H, $C_2H_5$ oder $-CH(CH_3)_2$ jeweils nur einmal vorhanden ist, und die Symbole $R^3$, $R^4$ und $R^7$ H oder $CH_3$ darstellen.

7. Verbindungen der allgemeinen Formel

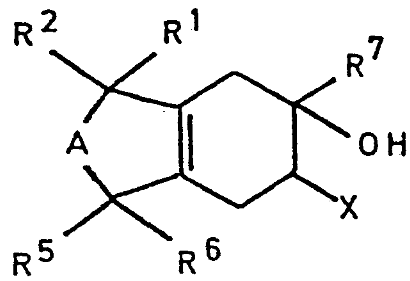

III

worin A für 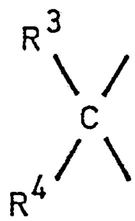 oder 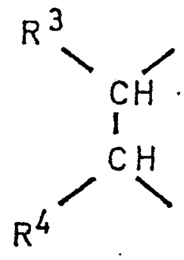 steht und die

Symbole $R^1$, $R^2$, $R^5$ und $R^6$ H, $CH_3$, $C_2H_5$ oder $-CH(CH_3)_2$ darstellen, wobei aber H, $C_2H_5$ oder $-CH(CH_3)_2$ jeweils nur einmal vorhanden ist, die Symbole $R^3$, $R^4$ und $R^7$ H oder $CH_3$ darstellen und X für Clor, Brom oder Jod steht.

8. Riech- und/oder Geschmackstoffkompositionen, gekennzeichnet durch einen Gehalt an einer Verbindung der allgemeinen Formel I gemäss Anspruch 1.

9. Verfahren zur Herstellung der Verbindungen der Formel I gemäss Anspruch 1, dadurch gekennzeichnet, dass man eine Verbindung der Formel

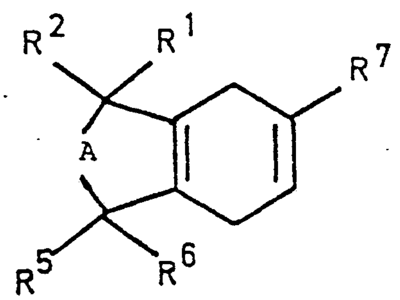

II

worin A und $R^1$ bis $R^7$ obige Bedeutung besitzen, epoxidiert.

10. Verwendung von Verbindungen der allgemeinen Formel I gemäss Anspruch 1 als Riech- und/oder Geschmackstoffe.

11. Verwendung von 6,7-Epoxy-1,1,3,4,4,6-hexamethyl-1,2,3,4,5,6,7,8-octahydronaphthalin als Riech- und/oder Geschmackstoff.

12. Verwendung von 6 7-Epoxy-1,1,4,4,6-pentamethyl-1,2,3,4,5,6,7,8-octahydronaphthalin als Riech- und/oder Geschmackstoff.

13. Verwendung von 5,6-Epoxy-1,1,2,3,3-pentamethyl-4,5,6,7-tetrahydroindan als Riech- und/oder Geschmackstoff.

**Claims**

1. Compounds of the general formula

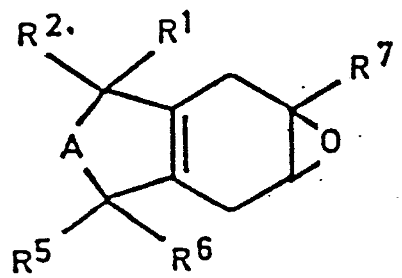

I

wherein A stands for 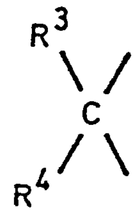 or 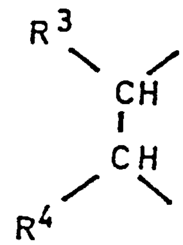 and

the symbols $R^1$, $R^2$, $R^5$ and $R^6$ represent H, $CH_3$, $C_2H_5$ or $-CH(CH_3)$, whereby in any given case only one H, $C_2H_5$ or $-CH(CH_3)_2$ is present, and the symbols $R^3$, $R^4$ and $R^7$ represent H or $CH_3$.

2. 6,7-Epoxy-1,1,3,4,4,6-hexamethyl-1,2,3,4,5,6,7,8-octahydronaphthalene.

3. 6,7-Epoxy-1,1,4,4,6-pentamethyl-1,2,3,4,5,6,7,8-octahydronaphthalene.

4. 5,6-Epoxy-1,1,2,3,3-pentamethyl-4,5,6,7-tetrahydro-indane.

5. A compound selected from: 5,6-Epoxy-1,1,3,3,5-pentamethyl-4,5,6,7-tetrahydroindane, 5,6-epoxy-1,1,2,3,3,5-hexamethyl-4,5,6,7-tetrahydroindane, 5,6-epoxy-3-ethyl-1,1,3,5-tetramethyl-4,5,6,7-tetrahydro-indane, 5,6-epoxy-1-isopropyl-2,3,3,5-tetramethyl-4,5,6,7-tetrahydroindane, 5,6-epoxy-1-isopropyl-2,2,3,3,5-penta-methyl-4,5,6.7-tetrahydroindane, 6,7-epoxy-1,1,4,4-tetra-methyl-1,2,3,4,5,6,7,8-octahydronaphthalene, 5,6-epoxy-3-ethyl-1,1,3,-trimethyl-4,5,6,7-tetrahydroindane, 6,7-epoxy-1,1,3,4,4-pentamethyl-1,2,3,4,5,6,7,8-octahydronaphthalene, 6,7-epoxy-1-ethyl-1,4,4,6-tetramethyl-1,2,3,4,5,6,7,8-octahydronaphthalene, 6,7-epoxy-1-iso-propyl-4,4,7-trimethyl-1,2,3,4,5,6,7,8-octahydronaphthalene, 6,7-epoxy-1-ethyl-1,4,4,7-tetramethyl-1,2,3,4,5,6,7,8-octahydronaphthalene.

6. Compounds of the general formula

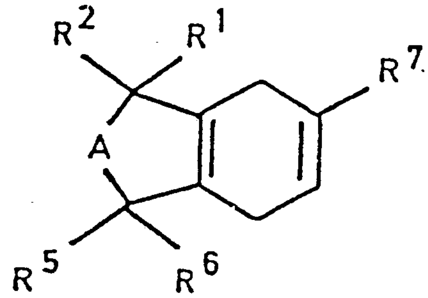

II

wherein A stands for 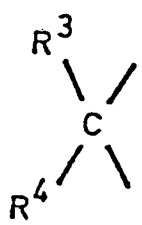 or 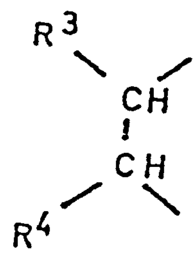 and

the symbols $R^1$, $R^2$, $R^5$ and $R^6$ represent H, $CH_3$, $C_2H_5$ or $-CH(CH_3)_2$, whereby in any given case only one H, $C_2H_5$ or $-CH(CH_3)_2$ is present, and the symbols $R^3$, $R^4$ and $R^7$ represent H or $CH_3$.

7. Compounds of the general formula

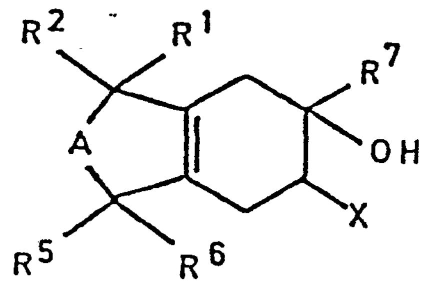

III

wherein A stands for 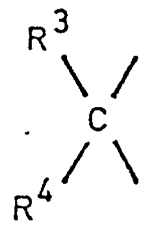 or 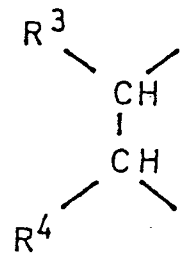 and

the symbols $R^1$, $R^2$, $R^5$ and $R^6$ represent H, $CH_3$, $C_2H_5$ or $-CH(CH^3)^2$, whereby in any given case only one H, $C^2H^5$ or $-CH(CH^3)^2$ is present, the symbols $R_3$, $R_4$ and $R_7$ represent H or $CH^3$ and X stands for chlorine, bromine or iodine.

8. Odorant and/or flavouring substance compositions, characterized by a content of a compound of general formula 1 in accordance with claim 1.

9. A process for the manufacture of the compounds of formula I in accordance with claim 1, characterized by epoxidizing a compound of the formula

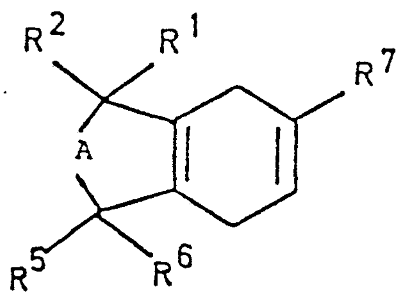

II

wherein A and $R^1$ to $R^7$ have the above significance.

10. The use of compounds of general formula I in accordance with claim 1 as odorant and/or flavouring substances.

11. The use of 6,7-epoxy-1,1,3,4,4,6-hexamethyl-1,2,3,4,5,6,7,8-octahydronaphthalene as an odorant and/or flavouring substance.

12. The use of 6,7-epoxy-1,1,4,4,6-pentamethyl-1,2,3,4,5,6,7,8-octahydronaphthalene as an odorant and/or flavouring substance.

13. The use of 5,6-epoxy-1,1,2,3,3-pentamethyl-4,5,6,7-tetrahydroindane as an odorant and/or flavouring substance.

**Revendications**

1. Composés de formule générale

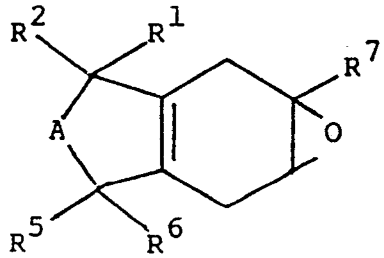

I

dans laquelle A représente 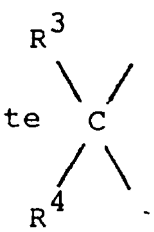 ou 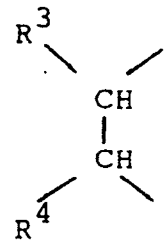

les symboles $R^1$, $R^2$, $R^5$ et $R^6$ représentent H, $CH_3$, $C_2H_5$ ou $-CH(CH_3)_2$, étant spécifié que H, $C_2H_5$ ou $-CH(CH_3)_2$ ne sont chacun présents qu'une fois, et les symboles $R^3$, $R^4$ et $R^7$ représentent H ou $CH_3$.

2. Le 6,7-époxy-1,1,3,4,4,6-hexaméthyl-1,2,3, 4,5,6,7,8-octahydronaphtalène.

3. Le 6,7-époxy-1,1,4,4,6-pentaméthyl-1,2,3,4, 5,6,7,8-octahydronaphtalène.

4. Le 5,6-époxy-1,1,2,3,3-pentaméthyl-4,5,6,7-tétrahydroindane.

5. Un composé choisi parmi: le 5,6-époxy-1,1,3,3,5-pentaméthyl-4,5,6,7-tétrahydro-indane, le 5,6-époxy-1,1,2,3,3,5-hexaméthyl-4,5,6,7-tétrahydroindane, le 5,6-époxy-3-éthyl-1,1,3,5-tétra-méthyl-4,5,6,7-tétrahydroindane, le 5,6-époxy-1-iso-propyl-2,3,3,5-tétraméthyl-4,5,6,7-tétrahydroindane, le 5,6-époxy-1-isopropyl-2,2,3,3,5-pentaméthyl-4,5,6,7-tétrahydroindane, le 6,7-époxy-1,1,4,4-tétraméthyl-1,2, 3,4,5,6,7,8-octahydronaphtalène, le 5,6-époxy-3-éthyl-1,1,3-triméthyl-4,5,6,7-tétrahydroindane, le 6,7-époxy-1,1,3,4,4-pentaméthyl-1,2,3,4,5,6,7,8-octahydronaphta-lène, le 6,7-époxy-1-éthyl-1,4,4,6-tétraméthyl-1,2,3, 4,5,6,7,8-octahydronaphtaléne, le 6,7-époxy-1-isopropyl-4,4,7-triméthyl-1,2,3,4,5,6,7,8-octahydronaphtalène, le 6,7-époxy-1-éthyl-1,4,4,7-tétraméthyl-1,2,3,4,5,6,7,8-octahydronaphtalène.

6. Composés de formule générale

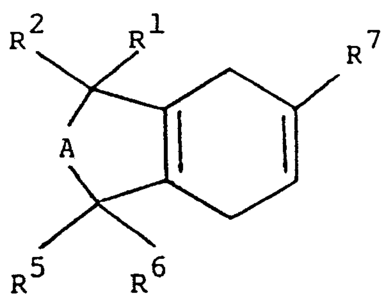

II

dans laquelle A représente 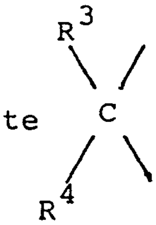 ou 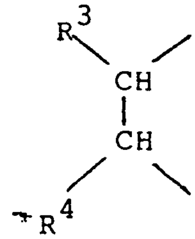

les symboles $R^1$, $R^2$, $R^5$ et $R^6$ représentent H, $CH_3$ $C_2H_5$ ou $-CH(CH_3)_2$, étant spécifié toutefois que H, $C_2H_5$ ou $-CH(CH_3)_2$ ne sont chacun présents qu'une fois, et les symboles $R^3$, $R^4$ et $R^7$ représentent H ou $CH_3$.

7. Composés de formule générale

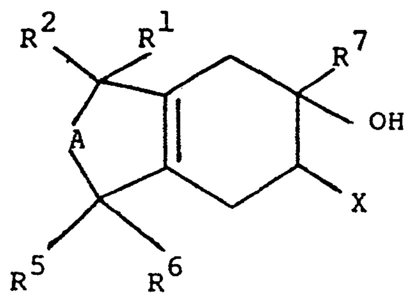

III

dans laquelle A représente

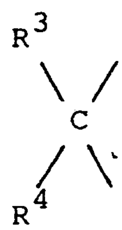

ou

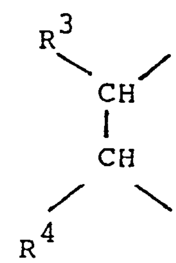

les symboles $R^1$, $R^2$, $R^5$ et $R^6$ représentent H, $CH_3$, $C_2H_5$ ou $-CH(CH_3)_2$, étant spécifié toutefois que H, $C_2H_5$ ou $-CH(CH_3)_2$ ne sont chacun présents qu'une fois, les symboles $R^3$, $R^4$ et $R^7$ représentent H ou $CH_3$ et X représente le chlore, le brome ou l'iode.

8. Compositions odorantes et/ou aromatisantes, caractérisées en ce qu'elles contiennent un composé de formule générale I selon la revendication 1.

9. Procédé de préparation des composés de formule générale I selon la revendication 1, caractérisé en ce qu'on époxyde un composé de formule

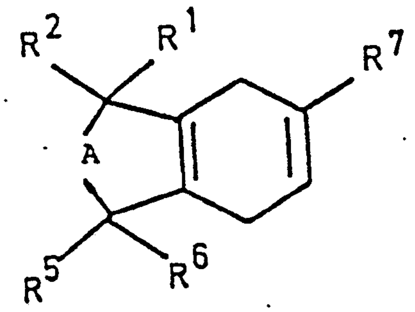

II

dans laquelle A et $R^1$ à $R^1$ ont les significations indiquées ci-dessus.

10. Utilisation des composés de formule générale I selon la revendication 1, en tant que substances odorantes et/ou aromatisantes.

11. utilisation du 6,7-époxy-1,1,3,4,4,6-hexaméthyl-1,2,3,4,5,6,7,8-octahydronaphtalène en tantque substance odorante et/ou aromatisante.

12. Utilisation du 6,7-époxy-1,1,4,4,6-penta-méthyl-1,2,3,4,5,6,7,8-octahydronaphtalène en tant que substance odorante et/ou aromatisante.

13. Utilisation du 5,6-époxy-1,1,2,3,3-penta-méthyl-4,5,6,7-tétrahydroindane en tant que substance odorante et/ou aromatisante.